# EUROPÄISCHE PATENTANMELDUNG

(11) **EP 3 940 570 A1**
(43) Veröffentlichungstag der Anmeldung: **19.01.2022**
(21) Anmeldenummer: 20185651.5
(22) Anmeldetag: 14.07.2020
(51) Int. Cl.: G06F 21/62, G16H 10/60, H04L 9/08, G06F 21/64

(54) **COMPUTERIMPLEMENTIERTES VERFAHREN ZUM EINLESEN UND SPEICHERN VON PATIENTENDATEN**

(71) Anmelder: Heil, Katharina, 8045 Graz (AT)
(72) Erfinder: Heil, Katharina, 8045 Graz (AT)
(74) Vertreter: Rothkopf, Ferdinand

(57) **Zusammenfassung**

Die Erfindung betrifft ein computerimplementiertes Verfahren zum Einlesen und Speichern von Patientendaten in einem Computersystem durch mehrere Nutzer mit den Schritten: Festlegen eines Studien-Schlüssels, Festlegen eines Nutzer-Schlüssels je Nutzers mittels des Studien-Schlüssels, Zuteilen der Nutzer-Schlüssel an die Nutzer sowie Einlesen von Patientendaten durch den einzelnen Nutzer mittels des jeweiligen Nutzer-Schlüssels, wobei die eingegebenen Patientendaten mittels des Nutzer-Schlüssels verschlüsselt im Computersystem gespeichert werden.

## Beschreibung

### Hintergrund der Erfindung

Die Erfindung betrifft ein computerimplementiertes Verfahren zum Einlesen und Speichern von Patientendaten in einem Computersystem durch mehrere Nutzer.

Bei der computergestützten Verarbeitung von medizinischen Patientendaten bestehen hinsichtlich des Datenschutzes besonders hohe Anforderungen und besonders strenge rechtliche Vorgaben. Bei den Patientendaten handelt es sich regelmäßig um personenbezogene Daten, für die ein besonders hohes Niveau an Datensicherheit zu gewährleisten ist.

Für Studien im medizinischen Bereich ist es erforderlich, dass solche Patientendaten auch über viele Standorte und dabei auch international verteilt erfasst sowie verarbeitet werden können. Zugleich sind umfangreiche Daten zu erfassen, um eine angemessene Dokumentation und Protokollierung solcher Studien zu gewährleisten.

Gerade bei der Erfassung bzw. dem Einlesen solcher Patientendaten im Rahmen von medizinischen Studien besteht noch viel Handlungsbedarf hinsichtlich der Digitalisierung solcher Prozesse.

### Zugrundeliegende Aufgabe

Der Erfindung liegt die Aufgabe zugrunde, ein computerimplementiertes Verfahren zum Einlesen und Speichern von Patientendaten in einem Computersystem durch mehrere Nutzer sowie ein zugehöriges Computersystem zu schaffen, mittels denen standortübergreifend Patientendaten auf besonders hohem Sicherheitsniveau eingelesen und gespeichert werden können.

### Erfindungsgemäße Lösung

Diese Aufgabe ist erfindungsgemäß mit einem computerimplementierten Verfahren zum Einlesen und Speichern von Patientendaten in einem Computersystem durch mehrere Nutzer geschaffen, bei dem die folgenden Schritte abgehandelt werden: Erstens Festlegen eines Studien-Schlüssels, zweitens Festlegen eines Nutzer-Schlüssels je Nutzer mittels des Studien-Schlüssels, drittens Zuteilen der Nutzer-Schlüssel an die Nutzer sowie viertens Einlesen von Patientendaten durch den einzelnen Nutzer mittels des jeweiligen Nutzer-Schlüssels, wobei die eingegebenen Patientendaten mittels des Nutzer-Schlüssels verschlüsselt im Computersystem gespeichert werden. Dabei sollen vorliegend gemäß der Erfindung mit dem Begriff "Studie" jede Art von Studie bzw. Untersuchung und auch andere Arten von Projekten mit inhaltlich zusammengehörigen Daten, also nicht nur medizinische oder etwa pharmazeutische Studien, verstanden werden.

Die Besonderheit der derartigen erfindungsgemäßen Vorgehensweise liegt darin, dass die Verschlüsselung beim Einlesen der Patientendaten auf einer zweistufigen Generierung von Schlüsseln, nämlich eines Studien-Schlüssels für die medizinische Studie selbst und eines Nutzer-Schlüssels für jeden der Nutzer beruht, wobei der jeweilige Nutzer-Schlüssel zusätzlich auf Basis bzw. Grundlage des Studien-Schlüssels erzeugt wird. Gemäß der Erfindung ist also der Studien-Schlüssel mit in dem Nutzer-Schlüssel verarbeitet bzw. integriert, so dass dieser jeweilige Nutzer-Schlüssel in verschlüsselter Weise auch die Information des Studien-Schlüssels enthält.

Aufgrund dieser besonderen Schlüssel-Systematik ist es gemäß der Erfindung möglich, dass die vom jeweiligen Nutzer eingegebenen Daten grundsätzlich sofort verschlüsselt werden und damit ausschließlich in verschlüsseltem Zustand im Computersystem gespeichert werden. Das Computersystem enthält also keinerlei nicht-verschlüsselte Information, wodurch das so genannte Konzept des Zero-Knowledge-Proof sichergestellt ist. Mit diesem Konzept ist also sichergestellt, dass der Anbieter bzw. Administrator des erfindungsgemäßen Computersystems selbst keinen Einblick in die gespeicherten Daten der Nutzer haben kann.

Darüber hinaus bietet diese Vorgehensweise gemäß der Erfindung den Vorteil, dass die Patientendaten weitestgehend unbedenklich auch verteilt an diversen Standorten und/oder auf verschiedenen Rechnern bzw. Servern gespeichert sowie selbst in so genannten Cloud-Speichern gehalten werden können, ohne dass ein Sicherheitsrisiko besteht.

Eine bevorzugte Ausführungsform des erfindungsgemäßen Verfahrens umfasst ferner die Schritte: Festlegen von Nutzer-Rechten zum Verarbeiten von gespeicherten Patientendaten in dem Nutzer-Schlüssel und Verarbeiten von gespeicherten Patientendaten im Computersystem durch einen Nutzer in Abhängigkeit von in dessen Nutzer-Schlüssel festgelegten Nutzer-Rechten zum Verarbeiten von gespeicherten Patientendaten. Der erfindungsgemäß vorgesehene Nutzer-Schlüssel enthält damit nicht nur zugleich die Verschlüsselungsinformation des Studien-Schlüssels sondern definiert als solcher auch noch Nutzer-Rechte, mittels denen ein Zugriff auf Patientendaten im Computersystem geregelt ist. Diese Rechte-Zuweisung kann dabei wiederum in sich verschlüsselt sein, insbesondere mittels des Studien-Schlüssels, so dass eine Manipulation dieser Rechtezuweisung durch Unbefugte ebenfalls weitestgehend ausgeschlossen ist.

Beim Festlegen der Nutzer-Rechte zum Verarbeiten von gespeicherten Patientendaten wird besonders bevorzugt festgelegt, dass der jeweilige Nutzer die von ihm selbst eingegebenen Daten stets verarbeiten darf. So kann sehr einfach der Zugriff auf "eigene" Daten des jeweiligen Nutzers sichergestellt werden.

Das erfindungsgemäße, computerimplementierte Verfahren umfasst gemäß derselben, oben erläuterten erfindungsgemäßen Idee ferner vorzugsweise die Schritte: Festlegen von Nutzer-Rechten zum Auslesen von gespeicherten Patientendaten in dem Nutzer-Schlüssel und Auslesen von gespeicherten Patientendaten im Computersystem durch einen Nutzer in Abhängigkeit von in dessen Nutzer-Schlüssel festgelegten Nutzer-Rechten zum Auslesen von gespeicherten Patientendaten. Dabei wird in ähnlicher Weise beim Festlegen der Nutzer-Rechte zum Auslesen von gespeicherten Patientendaten besonders vorteilhaft festgelegt, dass der jeweilige Nutzer die von ihm selbst eingegeben Daten stets auslesen darf.

Das computerimplementierte Verfahren gemäß der Erfindung kann darüber hinaus besonders sicher gestaltet werden, indem die gespeicherten Patientendaten fragmentiert im Computersystem gespeichert werden.

Ferner können mit der erfindungsgemäßen Vorgehensweise die gespeicherten Patientendaten vorzugsweise auch in einem Cloud-Speicher des Computersystems gespeichert werden.

Die Erfindung ist vorzugsweise auch auf ein derartiges computerimplementiertes Verfahren mit folgendem Schritt gerichtet: Verarbeiten von im Computersystem gespeicherten Patientendaten zu Ergebnisdaten, wobei die Ergebnisdaten mittels des Studien-Schlüssels und/oder mittels des jeweiligen Nutzer-Schlüssels verschlüsselt im Computer-System gespeichert werden. Die erfindungsgemäße, zweistufige und in sich verschachtelte Verschlüsselung wird damit vorzugsweise auch für die Verarbeitung von Patientendaten zu Ergebnisdaten und deren Speicherung verwendet.

Darüber hinaus betrifft die Erfindung vorteilhaft ein solches computerimplementiertes Verfahren, wobei Änderungen an Patientendaten und/oder Ergebnisdaten mittels einer Signierung mit dem Blockchain-Prinzip protokolliert werden. Änderungen an Daten können so revisionssicher rückverfolgt werden.

Schließlich ist die Erfindung auch auf ein Computersystem gerichtet, dass zum Ausführen eines solchen computerimplementierten Verfahrens gemäß der Erfindung angepasst ist.

### Kurzbeschreibung der Zeichnung

Nachfolgend wird ein Ausführungsbeispiel einer erfindungsgemäßen Lösung anhand der beigefügten schematischen Zeichnung näher erläutert. Es zeigt:
Fig. 1 ein Ausführungsbeispiels eines Computersystems gemäß der Erfindung und
Fig. 2 ein Ablaufschema eines Ausführungsbeispiels des Verfahrens gemäß der Erfindung.

### Detaillierte Beschreibung des Ausführungsbeispiels

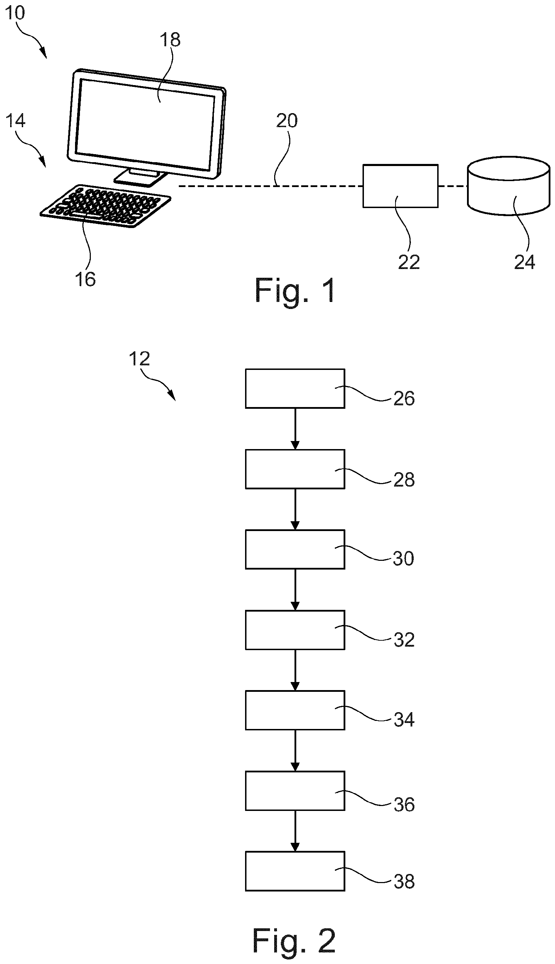

In der Fig. 1 ist ein Computersystem 10 veranschaulicht, mittels dem ein Verfahren 12 (siehe Fig. 2) durchzuführen ist.

Das Computersystem 10 umfasst eine Mehrzahl an Ein- und Ausgabeeinheiten 14, von denen beispielhaft nur eine dargestellt ist. Die Ein- und Ausgabeeinheit 14 umfasst in der Art eines Terminals zumindest eine Tastatur 16 sowie einen Bildschirm 18. Vorzugsweise umfasst die Ein- und Ausgabeeinheit 14 ferner eine Rechnereinheit bzw. Computereinheit (nicht näher veranschaulicht).

Angeschlossen ist die Ein- und Ausgabeeinheit 14 mittels einer Leitung 20, die drahtgebunden oder auch drahtlos gestaltet sein kann, an eine (weitere) Rechnereinheit 22. An diese Rechnereinheit 22 ist seinerseits eine Speichereinheit 24 betrieblich angekoppelt.

Mittels des Computersystems 10 ist das Verfahren 12 auszuführen, welches in Fig. 2 dargestellt ist. Bei dem Verfahren 12 wird zunächst in einem Schritt 26 von einem Administrator (nicht dargestellt) des Computersystems 10 ein Studien-Schlüssel in Gestalt einer ersten Kodierung bzw. eines ersten Codes über einen vom Computersystem 10 ausgeführten, ersten Algorithmus zur Schlüsselgenerierung festgelegt. Danach werden in einem Schritt 28 vom Administrator mittels des Computersystems 10 für mehrere Nutzer (nicht dargestellt) mehrere Nutzer-Schlüssel ebenfalls mittels eines zweiten Algorithmus festgelegt. Bei dieser Festlegung der Nutzer-Schlüssel wird der zuvor generierte Studien-Schlüssel innerhalb des zweiten Algorithmus berücksichtigt. Insbesondere werden die Nutzer-Schlüssel mittels des Studien-Schlüssels selbst verschlüsselt und/oder es wird in die Nutzer-Schlüssel die Information des Studien-Schlüssels integriert. Die Schlüssellänge beträgt dabei insbesondere 128 Bits bzw. 16 Bytes gemäß dem AES256-Standard.

Dann werden in einem Schritt 30 diese Nutzer-Schlüssel insbesondere in Form von QR-Codes per separater Schlüsselkarte dem jeweiligen Nutzer persönlich zugestellt. Der Nutzer kann sich dann nachfolgend in einem Schritt 32 mit seiner Ein- und Ausgabeeinheit und dem Nutzer-Schlüssel an dem Computersystem 10 anmelden. Der Nutzer kann dort dann in einem Schritt 34 Patientendaten einlesen und ggf. auch auslesen. Für das Einlesen werden die Patientendaten sofort in dem Schritt 34 bei der Eingabe mittels des Nutzer-Schlüssels verschlüsselt und in einem Schritt 36 ausschließlich verschlüsselt über die Leitung 20 an die Recheneinheit 22 bzw. die Speichereinheit 24 übermittelt. Alternativ können die Patientendaten auch derart verschlüsselt auf der Ein- und Ausgabeeinheit 14 selbst oder einem anderen Rechner bzw. Speicher, insbesondere einem Cloud-Rechner bzw. Cloud-Speicher vorgehalten werden.

Mittels der Ein- und Ausgabeeinheit 14 kann dann dieser Nutzer oder aber ein anderer Nutzer in einem Schritt 38 an einer anderen Ein- und Ausgabeeinheit 14 diese Patientendaten abrufen und/oder verarbeiten. Dabei ist der Zugriff auf die Patientendaten mittel des jeweiligen Nutzer-Schlüssels geregelt. Weil in dem Nutzer-Schlüssel jeweils zugleich auch die Information des Studien-Schlüssels enthalten ist, können die von einem ersten Nutzer eingegebenen und von diesem verschlüsselten Patientendaten oder zugehörige Ergebnisdaten auch von einem zweiten Nutzer in dem Schritt 38 ausgelesen und dabei entschlüsselt werden.

Abschließend sei angemerkt, dass sämtlichen Merkmalen, die in den Anmeldungsunterlagen und insbesondere in den abhängigen Ansprüchen genannt sind, trotz des vorgenommenen formalen Rückbezugs auf einen oder mehrere bestimmte Ansprüche, auch einzeln oder in beliebiger Kombination eigenständiger Schutz zukommen soll.

### Bezugszeichenliste

- 10: Computersystem
- 12: Verfahren
- 14: Ein- und Ausgabeeinheit
- 16: Tastatur
- 18: Bildschirm
- 20: Leitung
- 22: Recheneinheit
- 24: Speichereinheit
- 26: Schritt Erstellen Studien-Schlüssel
- 28: Schritt Erstellen Nutzer-Schlüssel
- 30: Schritt Zustellen Nutzer-Schlüssel
- 32: Schritt Anmelden Nutzer
- 34: Schritt Einlesen und Verschlüsseln Patientendaten
- 36: Schritt Übermitteln Patientendaten
- 38: Schritt Entschlüsseln und Auslesen der Patientendaten oder Ergebnisdaten

## Patentansprüche

1. Computerimplementiertes Verfahren (12) zum Einlesen und Speichern von Patientendaten in einem Computersystem (10) durch mehrere Nutzer mit den Schritten:
- Festlegen (26) eines Studien-Schlüssels,
- Festlegen (28) eines Nutzer-Schlüssels je Nutzers mittels des Studien-Schlüssels,
- Zuteilen (30) der Nutzer-Schlüssel an die Nutzer,
- Einlesen (34) von Patientendaten durch den einzelnen Nutzer mittels des jeweiligen Nutzer-Schlüssels, wobei die eingegebenen Patientendaten mittels des Nutzer-Schlüssels verschlüsselt im Computersystem (10) gespeichert werden.

2. Computerimplementiertes Verfahren nach Anspruch 1,
mit den Schritten:
- Festlegen von Nutzer-Rechten zum Verarbeiten von gespeicherten Patientendaten in dem Nutzer-Schlüssel und
- Verarbeiten von gespeicherten Patientendaten im Computersystem (10) durch einen Nutzer in Abhängigkeit von in dessen Nutzer-Schlüssel festgelegten Nutzer-Rechten zum Verarbeiten von gespeicherten Patientendaten.

3. Computerimplementiertes Verfahren nach Anspruch 2,
wobei beim Festlegen der Nutzer-Rechte zum Verarbeiten von gespeicherten Patientendaten festgelegt wird, dass der jeweilige Nutzer die von ihm selbst eingegeben Daten stets verarbeiten darf.

4. Computerimplementiertes Verfahren einem der Ansprüche 1 bis 3,
mit den Schritten:
- Festlegen von Nutzer-Rechten zum Auslesen (42) von gespeicherten Patientendaten in dem Nutzer-Schlüssel und
- Auslesen (42) von gespeicherten Patientendaten im Computersystem (10) durch einen Nutzer in Abhängigkeit von in dessen Nutzer-Schlüssel festgelegten Nutzer-Rechten zum Auslesen (42) von gespeicherten Patientendaten.

5. Computerimplementiertes Verfahren nach Anspruch 4,
wobei beim Festlegen der Nutzer-Rechte zum Auslesen (42) von gespeicherten Patientendaten festgelegt wird, dass der jeweilige Nutzer die von ihm selbst eingegeben Daten stets auslesen darf.

6. Computerimplementiertes Verfahren nach einem der Ansprüche 1 bis 5, wobei die gespeicherten Patientendaten fragmentiert im Computersystem (10) gespeichert werden.

7. Computerimplementiertes Verfahren nach einem der Ansprüche 1 bis 6, wobei die gespeicherten Patientendaten in einem Cloud-Speicher des Computersystems (10) gespeichert werden.

8. Computerimplementiertes Verfahren nach einem der Ansprüche 1 bis 7, mit dem Schritt:
Verarbeiten von im Computersystem (10) gespeicherten Patientendaten zu Ergebnisdaten,
wobei die Ergebnisdaten mittels des Studien-Schlüssels und/oder mittels des jeweiligen Nutzer-Schlüssels verschlüsselt im Computer-System (10) gespeichert werden.

9. Computerimplementiertes Verfahren nach einem der Ansprüche 1 bis 8, wobei Änderungen an Patientendaten und/oder Ergebnisdaten mittels einer Signierung mit dem Blockchain-Prinzip protokolliert werden.

10. Computersystem (10) angepasst zum Ausführen eines computerimplementierten Verfahrens nach einem der Ansprüche 1 bis 9.
